# EUROPEAN PATENT APPLICATION

(11) **EP 3 272 292 A1**
(43) Date of publication of application: **24.01.2018**
(21) Application number: 16187282.5
(22) Date of filing: 05.09.2016
(51) Int. Cl.: A61B 8/00

(54) **DEVICE FOR USE IN COMBINATION WITH AN ULTRASOUND IMAGING SYSTEM**

(30) Priority: 21.07.2016 US 201615215813
(71) Applicant: AmCad BioMed Corporation, Taipei 105 (TW)
(72) Inventor: Chen, Argon, 105 Taipei (TW); Lee, Yili, 105 Taipei (TW); Chen, Yu-Lun, 105 Taipei (TW); Huang, Kuo-Chen, 105 Taipei (TW)
(74) Representative: Becker Kurig Straus

(57) **Abstract**

The present invention provides a device for use in combination with an ultrasound imaging system including an ultrasonic probe (80), comprising a housing (10) including a main body (12) and a handle (14), the main body extending along a first axis from a first end (124) to a second end (126), and having an accommodating space (16); a holder (20) adapted for holding the ultrasonic probe, the holder being slidably movable within the accommodating space along the first axis with the handheld ultrasound probe held therein; a duct (30) for conveying conductive gel having an opening (32) at the first end of the main body; and a control means (40) adapted for controlling the supply of conductive gel from the opening of the duct.

## Description

### FIELD OF THE INVENTION

The present invention relates to a device for use in combination with an ultrasound imaging system including an ultrasonic probe, in particular a handheld device that holds the ultrasonic probe and allows convenient addition of conductive gel to the probe tip during the ultrasonic scanning process. The present invention also discloses a kit comprising said device.

### BACKGROUND OF THE INVENTION

US Patent No. 5,738,099 discloses a portable ultrasonic diagnostic apparatus comprising a pistol-shaped housing, and an LCD for displaying a result of ultrasonic diagnosis installed in a portion of the pistol-shaped housing.

China Utility Model Patent No. 201182611 Y discloses a handheld monitoring device for ultrasonic guide operation and anesthesia, comprising an ultrasonic probe and a handhold part, the ultrasonic probe being positioned at a front end of the handhold part. The monitoring device is characterized in that a display is configured at a rear end of the handhold part.

### SUMMARY OF THE INVENTION

In one aspect, the present invention provides a device for use in combination with an ultrasound imaging system including an ultrasonic probe. The device comprises a housing, a holder, a duct for conveying conductive gel, and a control means.

The housing includes a main body and a handle. The main body extends along a first axis from a first end to a second end. In addition, the main body has an accommodating space for receiving the ultrasonic probe and the holder.

The holder is adapted for holding the ultrasonic probe, and is slidably movable within the accommodating space along the first axis with the handheld ultrasound probe held therein.

The duct for conveying conductive gel has an opening or outlet at the first end of the housing.

The control means is adapted for controlling the supply of conductive gel from the opening or outlet of the duct.

In certain embodiments of the present invention, the ultrasound imaging system may further include a master device. According to one embodiment of the present invention, the device further comprises a communication module adapted to communicate with the master device.

In certain embodiments of the present invention, the ultrasonic probe may be an integrated handheld ultrasonic scanner. In such embodiments, the master device may be a device selected from the group consisting of a smartphone, a personal digital assistant, a tablet computer, a laptop computer, and a personal computer.

According one specific embodiment of the present invention, the device further comprises an adjustable display configured on the housing. The adjustable display may be detachably or securely mounted on the housing.

In certain preferred embodiments of the present invention, the control means is further adapted for controlling the movement of the holder within the accommodating space along the first axis.

According to the present invention, the device may further comprise a conductive gel source connected to the duct.

According to preferred embodiments of the present invention, the holder normally holds the ultrasonic probe in a first position such that the tip of the ultrasonic probe is exposed from the first end of the main body for performing the ultrasonic scanning. Further, the ultrasonic probe can be moved, by the holder along the first axis toward the second end of the main body, to a second position where its tip retreats to the accommodating space. In one embodiment, the movement of the holder along the first axis is controlled by the control means.

According to the present invention, the opening of the duct may be configured at a location adapted for supplying conductive gel to the tip of the ultrasonic probe when the ultrasonic probe is in the second position.

Preferably, the control means is adapted for synchronously moving the holder along the first axis and supplying conductive gel through the duct to the tip of the ultrasonic probe.

Preferably, after the conductive gel is added to the tip of the ultrasonic probe, the control means automatically moves the holder along the first axis toward the first end of the main body, such that the ultrasonic probe automatically returns to its first position.

In another aspect, the present invention provides a kit comprising a device as described above, and a base for said device.

According to certain embodiments of the present invention, the device further comprises a battery and the base is a charging base.

In one further aspect, the present invention provides a kit comprising a device as described above that has a communication module, and an intermediate apparatus. Such kit is designed for use in combination with a conventional ultrasonic imaging system whose master device is an ultrasound machine includes a console or control panel with physical buttons. The intermediate apparatus comprises a receiver for receiving a signal from the communication module, and an actuator for pressing a physical button of the console or control panel.

Preferably, the device further comprises a battery. According to the present invention, the actuator may be selected from the group consisting of electric magnets, electric motors, piezoelectric components, valves, pressure controllers, and combinations thereof.

These and other aspects will become apparent from the following description of the preferred embodiment taken in conjunction with the following drawings, although variations and modifications therein may be affected without departing from the spirit and scope of the novel concepts of the disclosure.

### BRIEF DESCRIPTION OF THE DRAWINGS

The foregoing summary, as well as the following detailed description of the invention, will be better understood when read in conjunction with the appended drawing. In the drawings:
Fig. 1 is a schematic view of a device according to one embodiment of the present invention.
Fig. 2A and Fig. 2B illustrates the relationship between the holder and the handheld ultrasound probe according to one embodiment of the present invention. Fig. 2C illustrates how the holder with the handheld ultrasound probe held therein is installed into the main body of the housing of a device according to one embodiment of the present invention.
Fig. 3 illustrates the duct for conveying conductive gel and the control means according to one embodiment of the present invention.
Fig. 4A and Fig. 4B are schematic views of a device according to one embodiment of the present invention, illustrating the movement of the handheld ultrasound probe between its first and second positions. Fig. 4C and Fig. 4D are schematic views of a device according to one embodiment of the present invention, illustrating the movement of the handheld ultrasound probe between its first and second positions.
Fig. 5A is a perspective view of a device according to one embodiment of the present invention. Fig. 5B is a perspective view of a device according to another embodiment of the present invention.
Fig. 6 is a perspective view of a kit according to one embodiment of the present invention.
Fig. 7 is a perspective view of a kit according to another embodiment of the present invention, used in combination with a conventional ultrasound imaging system.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

In one aspect, the present invention provides a device for use in combination with an ultrasound imaging system including an ultrasonic probe. The device comprises a housing, a holder, a duct for conveying conductive gel, and a control means. The housing includes a main body and a handle. The main body extends along a first axis from a first end to a second end, and the main body has an accommodating space for receiving the ultrasonic probe and the holder. The holder is adapted for holding the ultrasonic probe. Further, the holder is slidably movable along the first axis within the accommodating space with the handheld ultrasound probe held therein. The duct for conveying conductive gel has an opening or outlet at the first end of the main body. The control means is adapted for controlling the supply of conductive gel from the opening or outlet of the duct.

The device of the present invention is a handheld device. That is to say, the handle of the housing is adapted to be grasped by a human hand. In one embodiment of the present invention, the handle is connected to the second end of the main body. Preferably, the handle is pivotally connected to the main body and is adjustable and collapsible (by rotation with respect to the point where it is pivoted to the main body).

According to the present invention, the holder is preferably configured as a detachable element from the device. This is because to fit with different models of handheld ultrasound probes, there may be different models of holders correspondingly. Given a handheld ultrasound probe, a user may choose an appropriate or matched holder to hold the handheld ultrasound probe, and install the holder along with the probe into the accommodating space of the main body of the housing. In certain embodiments of the present invention, the holder takes the form of a casing of the handheld ultrasound probe. In one embodiment, the holder includes an upper casing and a lower casing which can engage with each other to form a casing that covers and holds the handheld ultrasound probe. In addition, the casing may have an exterior shape that fits with a (concave) track formed in the accommodating space, allowing the holder to slidably move along the first axis of the main body within the accommodating space. In another embodiment of the present invention, to enable the slidable movement the holder is connected to a wheel rotationally attached to a rail configured in the accommodating space.

According to the present invention, the device may further comprise a conductive gel source connected to the duct. In certain embodiments of the present invention, the conductive gel source is configured inside the handle. Preferably, the conductive gel source is a removable container containing conductive gel. More specifically, an accommodating space may be formed within the handle to receive the removable container.

According to the present invention, the control means may include a button configured on the housing, and a pump for conductive gel. In certain embodiments of the present invention, the button is slidably mounted on the housing, and the pump is a press pump. The press pump may be connected to the conductive gel source. The button is adapted to press on a piston of the press pump. Through pressing the button, the press pump pumps from the conductive gel source a certain amount of conductive gel into the duct, and in turn causes a certain amount of conductive gel to be supplied from the opening or outlet of the duct. Preferably, the press pump includes a spring for pushing the piston and the button back to their original positions. As an alternative, the pump may be an electronically controlled pump which can be controlled through the button.

In certain embodiments of the present invention, the ultrasound imaging system may further include a master device. According to one embodiment of the present invention, the device further comprises a communication module adapted to communicate with the master device.

For a conventional ultrasound imaging system, the master device is a so-called "ultrasound machine" which has a console or control panel with physical buttons. Examples for said conventional ultrasound imaging system include GE's LOGIQ E9 XDclear 2.0 ultrasound system, Philips' IE33 Ultrasound Machine, and Siemens' Acuson X150 Ultrasound Machine.

In some other embodiments of the present invention, the ultrasonic probe may be an integrated handheld ultrasonic scanner. For example, Philips' Lumify C5-2 broadband curved array transducer, SonoStar's Portable B Scanner, and a Clarius' L7 Linear Array Ultrasound Scanner. In such embodiments, the master device may be a device selected from the group consisting of a smartphone, a personal digital assistant, a tablet computer, a laptop computer, and a personal computer.

Optionally, the main body further includes a rear opening formed at its second end. The rear opening allows the wire of the ultrasonic probe to pass through.

According to certain embodiments of the present invention, the device further comprises a communication module adapted to communicate with the master device. The communication module may communicate with the mater device through a wire or wirelessly. The communication module functions, at least in part, to transmit certain control signals to the master device, and/or to receive image data from the master device for showing on the display. The communication module may include one or more push buttons for functional controls on ultrasound imaging. For example, the push buttons may be used to switch between different imaging modes (e.g. B-mode, US-mode, Doppler mode, etc.) and/or to enable/disable the image freezing or image recording functions of the master device.

According one specific embodiment of the present invention, the device further comprises an adjustable display configured on the housing. The adjustable display may be detachably or securely mounted on the housing. The adjustable display allows a medical personnel to conveniently observe the ultrasonic images when performing the scanning. The adjustable display may be adjustable in the sense that it can collapse toward the main body of the housing, either for storage or for convenience of observation.

In the cases that the ultrasonic probe is an integrated handheld ultrasonic scanner, the device of the present invention may further comprise a dock, configured on the housing, for the smartphone, personal digital assistant, or tablet computer, which partially serves as the display. The dock may also be adjustable in the sense that a user can adjust the angle of the screen of the smartphone, personal digital assistant, or tablet computer.

In certain preferred embodiments of the present invention, the control means is further adapted for controlling the movement of the holder within the accommodating space along the first axis.

According to preferred embodiments of the present invention, the holder normally holds the ultrasonic probe in a first position such that the tip of the ultrasonic probe is exposed from the first end of the main body for performing the ultrasonic scanning. Further, the ultrasonic probe can be moved, by the holder along the first axis toward the second end of the main body, to a second position where its tip retreats to the accommodating space. In one embodiment, the movement of the holder along the first axis is controlled by the control means.

According to the present invention, the opening of the duct may be configured at a location adapted for supplying conductive gel to the tip of the ultrasonic probe when the ultrasonic probe is in the second position.

Preferably, the control means is adapted for synchronously moving the holder along the first axis and supplying conductive gel through the duct to the tip of the ultrasonic probe.

Preferably, after the conductive gel is added to the tip of the ultrasonic probe, the control means automatically moves the holder along the first axis toward the first end of the main body, such that the ultrasonic probe automatically returns to its first position.

As mentioned above, for gel supply, the control means may include a button slidably mounted on the housing, and a press pump for conductive gel. According to preferred embodiments of the present invention, the button is mechanically coupled to the holder such that the holder with the ultrasonic probe held therein can be moved along the first axis toward the second end of the main body by pressing the button. Accordingly, the button of the control means can be used to synchronously move the holder along the first axis and to supply conductive gel through the duct to the tip of the ultrasonic probe. In addition, the control means may further include a spring configured between the holder and the main body, such that after the conductive gel is added to the tip of the ultrasonic probe and the button is released, the spring automatically moves the holder along the first axis toward the first end of the main body, bring the ultrasonic probe back to its first position.

In another aspect, the present invention provides a kit comprising a device as described above, and a base for said device.

According to certain embodiments of the present invention, the device further comprises a battery and the base is a charging base.

In one further aspect, the present invention provides a kit comprising a device as described above that has a communication module, and an intermediate apparatus. Such kit is designed for use in combination with a conventional ultrasonic imaging system whose master device is an ultrasound machine includes a console or control panel with physical buttons. The intermediate apparatus comprises a receiver for receiving a signal from the communication module, and an actuator for pressing a physical button of the console or control panel. The physical buttons are used to, for example, switch between different imaging modes (e.g. B-mode, US-mode, Doppler mode, etc.) and/or to enable/disable the image freezing or image recording functions of the master device.

Preferably, the device further comprises a battery. According to the present invention, the actuator may be selected from the group consisting of electric magnets, electric motors, piezoelectric components, valves, pressure controllers, and combinations thereof.

Certain preferred embodiments of the present invention are described below.

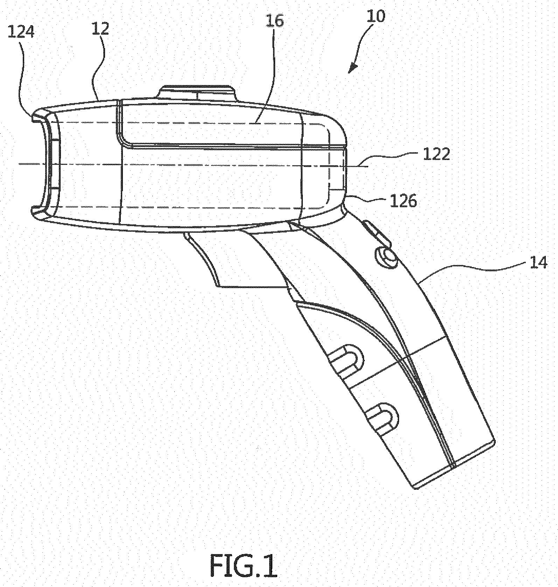

With reference to Figs. 1-4, provided is a device for use in combination with an ultrasound imaging system including an ultrasonic probe 80, comprising a housing 10, a holder 20, a duct 30 for conveying conductive gel, and a control means 40. The housing 10 includes a main body 12 and a handle 14, wherein the main body 12 extends along a first axis 122 from a first end 124 to a second end 126, and the main body 12 has an accommodating space 16. The handle 14 is connected to the second end 126 of the main body 12. The holder 20 is adapted for holding the ultrasonic probe 80, and is slidably movable within the accommodating space 16 along the first axis 122 with the handheld ultrasound probe 80 held therein. The duct 30 has an opening 32 at the first end 124 of the main body 12. The control means 40 is adapted for controlling the supply of conductive gel from the opening 32 of the duct 30.

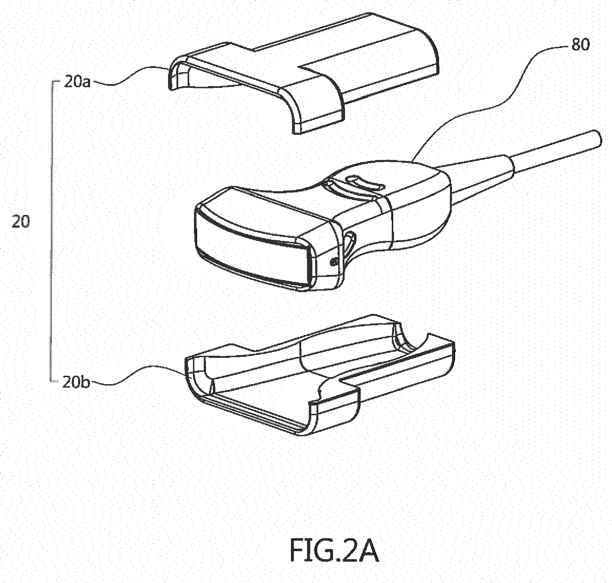

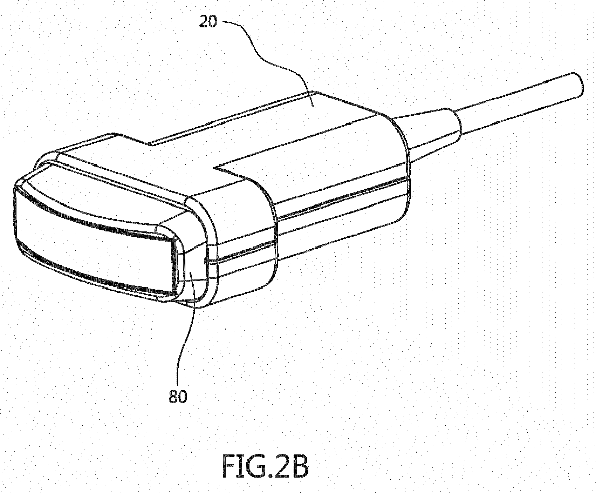

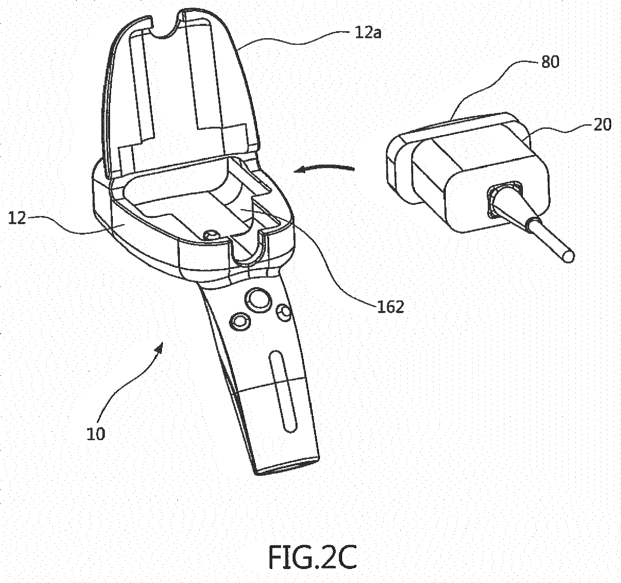

As shown in Figs. 2A and 2B, the holder 20 is a detachable element from the device. Further, the holder includes an upper casing 20a and a lower casing 20b which can engage with each other to form a casing that covers and holds the handheld ultrasound probe 80. Said casing has an exterior shape that fits with a concave track 162 formed in the accommodating space 16, allowing the holder 20 to slidably move along the first axis 122 of the main body 12 within the accommodating space 16. A user may combine the upper casing 20a and the lower casing 20b together with the ultrasonic probe 80 clamped therebetween, and then install the holder 20 along with the ultrasonic probe 80 into the main body 12 of the housing 10. The main body 12 may comprises an openable cover 12a which can be opened for ease of the installation.

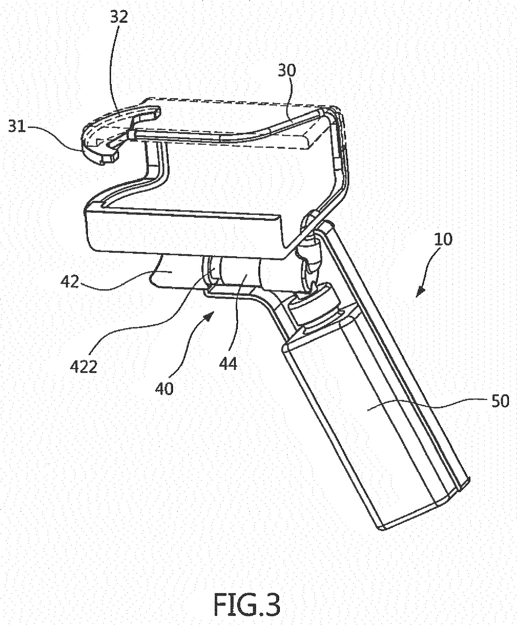

As illustrated in Fig. 3, the duct 30 further comprises a dispersion head 31 for average supply of conductive gel. An arc shaped opening 32 may be configured on the dispersion head 31. On the other hand, the control means 40 includes a button 42 slidably mounted on the housing 10, and a press pump 44 for conductive gel, connected to a conductive gel source 50. The button 42 is adapted to press on a piston 442 of the press pump 44. Through pressing the button 42, the press pump 44 pumps from the conductive gel source 50 a certain amount of conductive gel into the duct 30, and in turn causes a certain amount of conductive gel to be supplied from the opening 32. The press pump may further include a spring (not shown) for pushing the piston 442 and the button 42 back to their original positions.

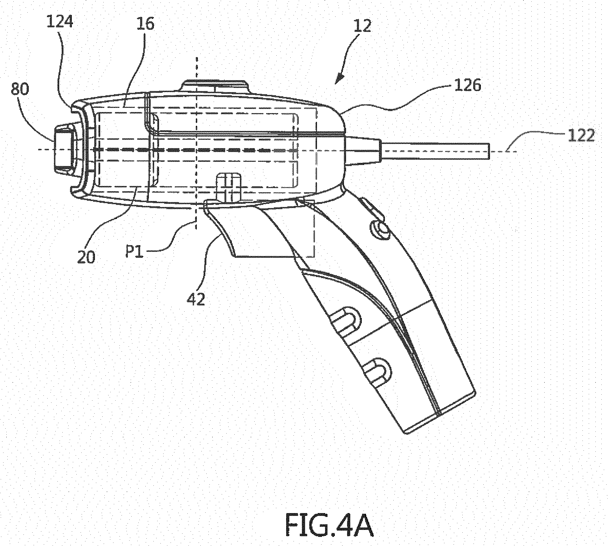

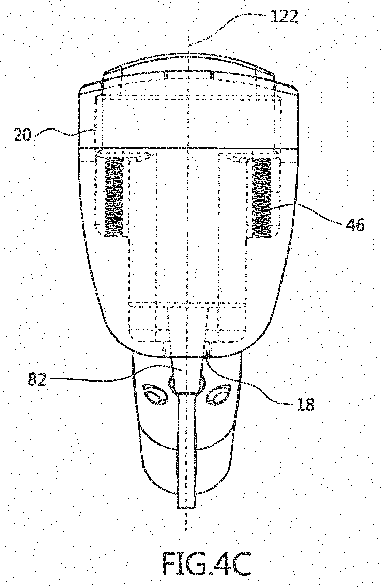

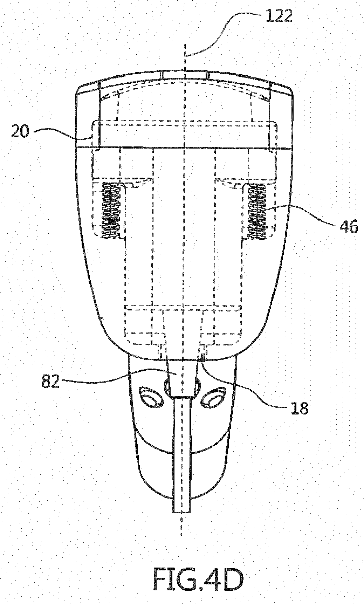

Referring to Fig. 3 as well as Fig. 4A-4D, the holder 20 normally holds the ultrasonic probe 80 in a first position P1 such that the tip of the ultrasonic probe 80 is exposed from the first end 124 of the main body 12 for performing the ultrasonic scanning. The ultrasonic probe 80 can be moved by the holder 20 along the first axis 122 toward the second end 126 of the main body 12, to a second position P2 where its tip retreats to the accommodating space 16. The movement of the holder 20 along the first axis 122 is controlled by the button 42 of the control means 40. The button 42 is mechanically coupled to the holder 20 such that the holder 20 with the ultrasonic probe 80 held therein can be moved along the first axis 122 toward the second end 126 of the main body 12 by pressing the button 42. Accordingly, the button 42 of the control means 40 can be used to synchronously move the holder 20 along the first axis 122 and to supply conductive gel through the duct 30 to the tip of the ultrasonic probe 80. Furthermore, the control means further includes a spring 46 configured between the holder 20 and the main body 12, such that after the conductive gel is added to the tip of the ultrasonic probe 80 and the button 42 is released, the spring 46 automatically moves the holder 20 along the first axis 122 toward the first end 124 of the main body 12, bring the ultrasonic probe 80 back to its first position P1. The main body 12 may further include a rear opening 18 formed at its second end 126. The rear opening 18 allows the wire 82 of the ultrasonic probe 80 to pass through.

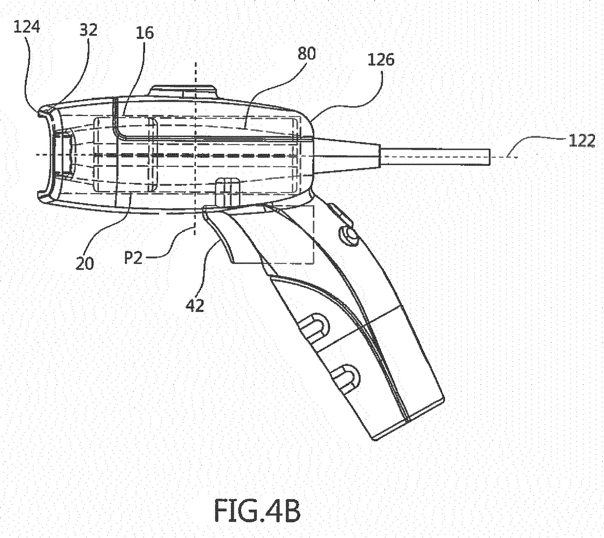

As shown in Fig. 4B, the opening 32 of the duct 30 may be configured at a location adapted for supplying conductive gel to the tip of the ultrasonic probe 80 when the ultrasonic probe 80 is in the second position P2.

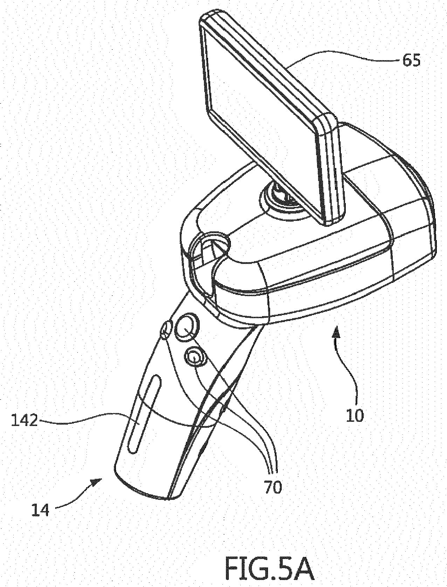

Referring to Fig. 5A, shown is a device according to another embodiment of the present invention, the device comprising an adjustable display 65 configured on the housing 10. The adjustable display 65 may be detachably or securely mounted on the housing 10. In this embodiment, the conductive gel is stored in and supplied from a transparent container (not shown) configured within the handle 14. The handle 14 further comprises an observation window 142 allowing a user to check the remaining amount of the conductive gel.

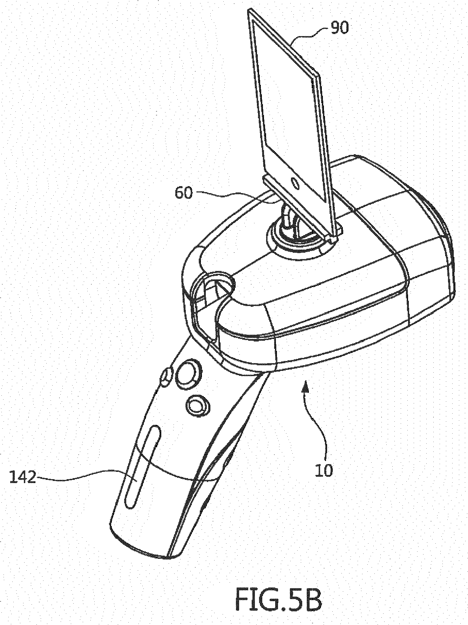

Fig. 5B illustrates a device according to another embodiment of the present invention, wherein the ultrasonic probe is an integrated handheld ultrasonic scanner. The device may further comprise a dock 60 configured on the housing 10. A master device 90, which is a smartphone in this embodiment, can be fixed on the dock 60. The master device 90 may partially serves as a display.

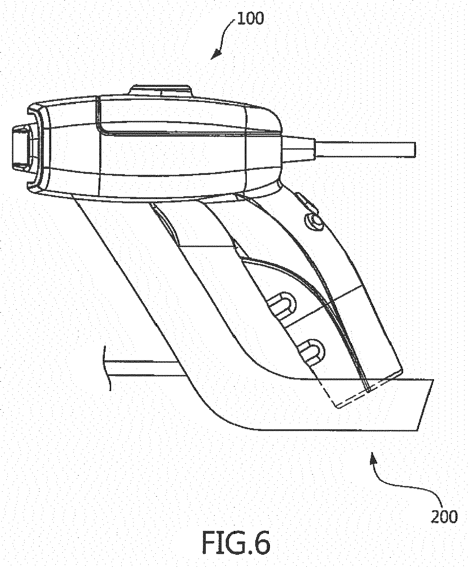

Referring to Fig. 6, provided is a kit comprising a device 100 of the present invention which includes a battery (not shown), and a charging base 200 for said device. The device 100 can rest on the charging base 200 when not in use or for charging.

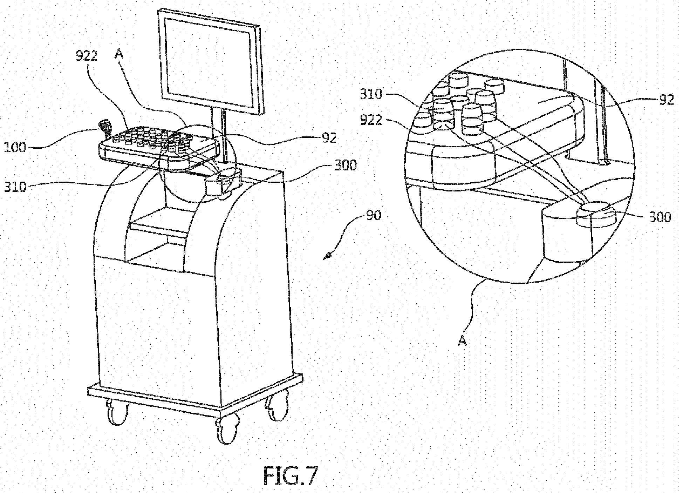

With reference to Fig. 7, shown is a kit comprising a device 100 of the present invention that has a communication module (not shown) including one or more pushing buttons 70, and an intermediate apparatus 300. This kit can be used in combination with a conventional ultrasonic imaging system 90 whose master device is an ultrasound machine includes a console 92 with physical buttons 922. The intermediate apparatus 300 comprises a receiver (not shown) for receiving a signal from the communication module, and an actuator 310 for pressing a physical button 922 of the console 92. By pressing a specific pushing button 70, the communication module sends a corresponding signal to the receiver to activate a corresponding actuator 310 for pressing the physical button 922 on which said actuator 310 is disposed.

It is believed that a person of ordinary knowledge in the art where the present invention belongs can utilize the present invention to its broadest scope based on the descriptions herein with no need of further illustration. Therefore, the descriptions and claims as provided should be understood as of demonstrative purpose instead of limitative in any way to the scope of the present invention.

## Claims

1. A device for use in combination with an ultrasound imaging system including a handheld ultrasonic probe, comprising:
a housing including a main body and a handle, the main body extending along a first axis from a first end to a second end, and having an accommodating space;
a holder adapted for holding the ultrasonic probe, the holder being slidably movable within the accommodating space along the first axis with the handheld ultrasound probe held therein;
a duct for conveying conductive gel having an opening at the first end of the main body; and
a control means adapted for controlling the supply of conductive gel from the opening of the duct.

2. The device of claim 1, wherein the ultrasound imaging system further includes a master device.

3. The device of claim 2, further comprising a communication module adapted to communicate with the master device.

4. The device of claim 2, wherein the ultrasonic probe is an integrated handheld ultrasonic scanner, and the master device is a device selected from the group consisting of a smartphone, a personal digital assistant, a tablet computer, a laptop computer, and a personal computer.

5. The device of claim 1, further comprising an adjustable display configured on the housing, the adjustable display being detachably or securely mounted on the housing.

6. The device of claim 4, further comprising a dock for the smartphone, personal digital assistant, or tablet computer, wherein the dock is configured on the housing.

7. The device of claim 1, wherein the control means is further adapted for controlling the movement of the holder along the first axis.

8. The device of claim 1, further comprising a conductive gel source connected to the duct.

9. The device of claim 8, wherein the conductive gel source is configured inside the handle.

10. The device of claim 9, wherein the conductive gel source is a removable container containing conductive gel.

11. The device of claim 1, wherein the holder holds the ultrasonic probe in a first position such that the tip of the ultrasonic probe is exposed from the first end of the main body, and the ultrasonic probe is movable, by the holder along the first axis toward the second end of the main body, to a second position where its tip retreats to the accommodating space.

12. The device of claim 11,
wherein the movement of the holder along the first axis is controlled by the control mean; preferably wherein the control means is adapted for synchronously moving the holder along the first axis and supplying conductive gel through the duct to the tip of the ultrasonic probe; or
wherein the opening of the duct is configured at a location adapted for supplying conductive gel to the tip of the ultrasonic probe when the ultrasonic probe is in the second position, preferably wherein, after the conductive gel is added to the tip of the ultrasonic probe, the control means automatically moves the holder along the first axis toward the first end of the main body, such that the ultrasonic probe automatically returns to its first position.

13. A kit comprising:
a device according to claim 1; and
a base for the device;
preferably wherein the device further comprises a battery and the base is a charging base.

14. A kit comprising:
device according to claim 3, wherein the master device includes a console; and
an intermediate apparatus comprising: a receiver for receiving a signal from the communication module; and an actuator for pressing a physical button of the console.

15. The kit according to claim 14,
wherein the device further comprises a battery; or
wherein the actuator is selected from the group consisting of electric magnets, electric motors, piezoelectric components, valves, pressure controllers, and combinations thereof.
